# EUROPÄISCHE PATENTSCHRIFT

(11) **EP 2 943 464 B1**
(45) Veröffentlichungstag und Bekanntmachung des Hinweises auf die Patenterteilung: **01.03.2017**
(21) Anmeldenummer: 13811566.2
(22) Anmeldetag: 20.12.2013
(51) Int. Cl.: C07C 211/27, C08G 59/50, C08G 59/56, C08L 63/00, C09D 163/00

(54) **AMIN FÜR EMISSIONSARME EPOXIDHARZ-PRODUKTE**
AMINE FOR LOW-EMISSION EPOXY RESIN PRODUCTS
AMINE POUR RÉSINES ÉPOXY À FAIBLES ÉMISSIONS

(30) Priorität: 08.01.2013 EP 13150534
(43) Veröffentlichungstag der Anmeldung: 18.11.2015
(73) Patentinhaber: Sika Technology AG, 6340 Baar (CH)
(72) Erfinder: KASEMI, Edis, CH-8046 Zürich (CH); KRAMER, Andreas, CH-8006 Zürich (CH); STADELMANN, Ursula, CH-8046 Zürich (CH); BURCKHARDT, Urs, CH-8049 Zürich (CH)
(74) Vertreter: Sika Patent Attorneys
(86) Internationale Anmeldenummer: PCT/EP2013/077704
(87) Internationale Veröffentlichungsnummer: WO 2014/108306

(56) Entgegenhaltungen:
- EP-A1- 2 159 218
- EP-A2- 1 188 740
- US-A- 5 739 209

## Beschreibung

### Technisches Gebiet

Die Erfindung betrifft das Gebiet der Amine, Härter für Epoxidharze, Epoxidharz-Produkte sowie deren Verwendung, insbesondere als Beschichtungen, Beläge und Anstriche.

### Stand der Technik

Für Beschichtungszwecke geeignete Epoxidharz-Produkte sollen eine möglichst niedrige Viskosität aufweisen, damit sie bei Umgebungstemperatur gut verarbeitbar und selbstverlaufend sind. Weiterhin sollen sie möglichst schnell und störungsfrei aushärten, auch bei feucht-kalten Bedingungen, und dabei eine ebenmässige Oberfläche ohne Trübungen, Flecken oder Krater ausbilden. Schliesslich soll eine ausgehärtete Beschichtung eine hohe Härte bei geringer Sprödigkeit besitzen, um mechanischer Beanspruchung möglichst gut zu widerstehen. Für optisch anspruchsvolle Anwendungen, beispielsweise Deckbeläge von Fussböden, soll eine Beschichtung ausserdem eine möglichst geringe Neigung zum Vergilben unter Lichteinfluss aufweisen.

Um diese Eigenschaften zu erreichen, werden in Epoxidharz-Beschichtungen nach dem Stand der Technik üblicherweise Verdünner eingesetzt. Die Verdünner verbessern die Verarbeitbarkeit, reduzieren die Sprödigkeit der Beschichtung und verbessern die Oberflächenqualität, indem sie das Auftreten von Blushing-Effekten vermindern. Als "Blushing-Effekte" werden bei der Aushärtung auftretende Oberflächenmängel wie Trübungen, Flecken, Rauheit und Klebrigkeit bezeichnet, welche durch Salzbildung ("Blushing") von Aminen mit Kohlendioxid (CO₂) aus der Luft verursacht werden und besonders bei hoher Luftfeuchtigkeit und tiefen Temperaturen auftreten.

Die üblicherweise eingesetzten Verdünner, insbesondere Benzylalkohol sowie Glykole und Alkylphenole, sind gegenüber Epoxidharzen bei Raumtemperatur unreaktiv und werden daher bei der Aushärtung nicht in die Harzmatrix eingebaut. Heutzutage werden aber zunehmend emissionsarme Produkte gewünscht, die nach der Aushärtung einen geringen Gehalt von durch Verdampfungs- oder Diffusionsprozesse freisetzbaren Substanzen aufweisen. Für emissionsarme Epoxidharz-Produkte können nicht einbaubare Verdünner deshalb nur in sehr geringer Menge oder gar nicht verwendet werden.

Eine andere Möglichkeit, Epoxidharze zu verdünnen, besteht im Einsatz von erhöhten Mengen an kleinen primären Aminen in der Härterkomponente. Solche Amine, wie beispielsweise Diethylentriamin, Isophorondiamin oder Xylylendiamin, sind aber geruchsintensiv, stark haut- und augenreizend sowie sensibilisierend, und sie führen verstärkt zu Blushing-Effekten.

US 5,426,157 beschreibt Epoxidharz-Zusammensetzungen enthaltend N,N'-dimethylierte Diamine. US 5,739,209 beschreibt monoalkylierte Diamine ausgehend von 2-Methyl-1,5-pentandiamin als Härter für Epoxidharze mit erhöhter Flexibilität und Beständigkeit. Die beschriebenen Diamine verdünnen emissionsarme Epoxidharz-Beschichtungen aber nur ungenügend.

EP 2,159,218 A offenbart Amine-Zusammensetzungen sowie Amine-Epoxy-Zusammensetzungen, die N,N'-Dimethyl-meta-xylylendiamine enthalten.

### Darstellung der Erfindung

Aufgabe der vorliegenden Erfindung ist es daher, ein Amin zur Verfügung zu stellen, welches in emissionsarmen, raumtemperaturhärtenden Epoxidharz-Produkten, welche insbesondere für Beschichtungszwecke geeignet sind, vorteilhaft verwendet werden kann, indem es das Epoxidharz sehr gut verdünnt, eine rasche und störungsfreie Aushärtung ermöglicht und die Sprödigkeit des ausgehärteten Harzes reduziert.

Überraschenderweise wurde gefunden, dass das Amin der Formel (I) diese Aufgabe sehr gut löst. Es ist geruchsarm und wenig flüchtig, trotzdem sehr niedrigviskos und verdünnt Epoxidharz-Beschichtungen überraschend gut, ohne die Aushärtungsgeschwindigkeit allzu stark zu verlangsamen oder Blushing-Effekte zu bewirken. Bei der Aushärtung wird es in die Harzmatrix eingebaut und trägt bei hoher Härte sehr effektiv zur Reduktion der Sprödigkeit bei. Im Gegensatz zu ähnlichen Aminen, wie 1,3-Bis(benzylaminomethyl)benzol oder 1,3-Bis(2-phenylethylaminomethyl)benzol, bewirkt es überraschenderweise keine verstärkte Vergilbung der ausgehärteten Beschichtung.

Mit dem Amin der Formel (I) sind Härter für emissionsarme, bei Raumtemperatur härtende Epoxidharze zugänglich, welche die Bedingungen für Öko-Gütesiegel, beispielsweise nach Emicode (EC1 Plus), AgBB, DIBt, Der Blaue Engel, AFSSET, RTS (M1) und US Green Building Council (LEED), erfüllen und gleichzeitig hohen Ansprüchen bezüglich Verarbeitungs- und Gebrauchseigenschaften genügen.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Amin der Formel (I).

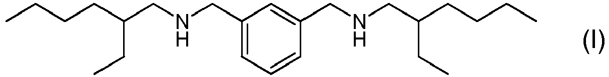

Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten.

Als "Aminwasserstoff" werden die Wasserstoffatome von primären und sekundären Aminogruppen bezeichnet.

Als "Aminwasserstoff-Equivalentgewicht" wird der Gewichtsanteil eines Härters oder eines Amins pro im Härter oder im Amin vorhandenem Aminwasserstoff bezeichnet.

Als "nicht einbaubarer Verdünner" wird eine in einem Epoxidharz lösliche und dessen Viskosität senkende Substanz bezeichnet, welche bei der Aushärtung des Epoxidharzes nicht kovalent in die Harzmatrix eingebaut wird.

Mit dem Begriff "Viskosität" wird im vorliegenden Dokument die dynamische Viskosität oder Scherviskosität bezeichnet, welche durch das Verhältnis zwischen der Schubspannung und der Scherrate (Geschwindigkeitsgefälle) definiert ist und wie in den Ausführungsbeispielen beschrieben bestimmt wird.

Beim Amin der Formel (I) handelt es sich um 1,3-Bis(2-ethylhexylaminomethyl)benzol.

Das Amin der Formel (I) kann besonders vorteilhaft durch reduktive Alkylierung von 1,3-Bis(aminomethyl)benzol (meta-Xylylendiamin oder MXDA) mit 2-Ethylhexanal erhalten werden. Die reduktive Alkylierung kann direkt mit molekularem Wasserstoff oder indirekt durch Wasserstoff-Transfer von anderen Reagentien erfolgen. Bevorzugt wird molekularer Wasserstoff verwendet. Dabei werden die Bedingungen vorteilhaft so gewählt, dass einerseits die primären Aminogruppen mit guter Selektivität einfach alkyliert werden und andererseits der Benzolring nicht hydriert wird.

Bevorzugt wird bei einem Wasserstoff-Druck von 5 bis 100 bar, einer Temperatur von 40 bis 120 °C und in Anwesenheit eines geeigneten Katalysators gearbeitet. Als Katalysator bevorzugt sind Palladium auf Kohle (Pd/C), Platin auf Kohle (Pt/C), Adams-Katalysator und Raney-Nickel, insbesondere Palladium auf Kohle und Platin auf Kohle.

Die Herstellung des Amins der Formel (I) durch reduktive Alkylierung auf die beschriebene Weise ist für die Verwendung als Bestandteil von Härtern für Epoxidharze besonders vorteilhaft, da primäre Aminogruppen mit guter Selektivität einfach alkyliert werden, während sekundäre Aminogruppen kaum weiter alkyliert werden. Das Produkt aus dem beschriebenen Herstellverfahren kann deshalb ohne weitere Aufbereitung zur Aushärtung von Epoxidharzen in der beschriebenen Weise verwendet werden.

Ein weiterer Gegenstand der Erfindung ist somit ein Verfahren zur Herstellung des Amins der Formel (I) durch reduktive Alkylierung von 1,3-Bis(aminomethyl)benzol mit 2-Ethylhexanal und Wasserstoff.

Bevorzugt werden 2-Ethylhexanal und 1,3-Bis(aminomethyl)benzol im Molverhältnis im Bereich von 1.4/1 bis 2.4/1, insbesondere 1.6/1 bis 2.2/1, eingesetzt. Ein Molverhältnis von < 1.4/1 kann bei der Anwendung im Epoxidharz-Härter zu unbefriedigenden Ergebnissen in Bezug auf Verdünnungswirkung und Blushing führen, während ein Molverhältnis > 2.4/1 eine aufwendige Nachreinigung erfordert.

Besonders bevorzugt ist ein Molverhältnis von ungefähr 2/1. Dabei ist das Amin der Formel (I) ohne zusätzliche Aufarbeitung in hoher Reinheit erhältlich und bewirkt als Bestandteil von Epoxidharz-Härtern eine hervorragende verdünnende und die Sprödigkeit reduzierende Wirkung.

Besonders bevorzugt ist weiterhin ein Molverhältnis von ungefähr 1.6/1. Das resultierende Reaktionsgemisch enthält neben dem Amin der Formel (I) auch erhebliche Anteile von N-2-Ethylhexyl-1,3-bis(aminomethyl)benzol. Es hat als Bestandteil von Epoxidharz-Härtern eine gute verdünnende Wirkung, eine moderate die Sprödigkeit reduuzierende Wirkung und ermöglicht eine besonders schnelle Aushärtung.

Das Amin der Formel (I) lässt sich auch auf andere Weise als durch reduktive Alkylierung erhalten, insbesondere durch Umsetzung von 1,3-Bis(aminomethyl)benzol mit 2-Ethylhexylchlorid oder 2-Ethylhexylbromid in einem geeigneten Verhältnis. Dabei entstehen Reaktionsgemische, welche typischerweise einen erheblichen Anteil an doppelt alkylierten Aminogruppen aufweisen.

Das Amin der Formel (I) ist eine wenig flüchtige, geruchsarme Substanz von sehr niedriger Viskosität. Es weist eine so geringe Reaktivität gegenüber CO₂ auf, dass es - im Gegensatz zu vielen aus dem Stand der Technik bekannten Aminen - an der Luft weder zur Bildung von Krusten noch zu Ausfällungen oder Viskositätserhöhungen neigt. Es zeigt eine ausgezeichnete Verträglichkeit mit anderen Aminen und mit Epoxidharzen. Trotz der Anwesenheit des aromatischen Rings führt es in der ausgehärteten Epoxidharz-Beschichtung überraschenderweise nicht zu verstärkter Vergilbung unter Lichteinfluss.

Ein weiterer Gegenstand der Erfindung ist die Verwendung des Amins der Formel (I) als Verdünner, insbesondere in Härtern für Epoxidharze.

Das Amin der Formel (I) weist ein vergleichsweise hohes Aminwasserstoff-Equivalentgewicht auf. Dadurch kann es in erheblicher Menge in Härtern für Epoxidharze eingesetzt werden und diese verdünnen, ohne die Aushärtungsreaktion allzu stark zu beeinflussen.

Im Fall von Härtern für Epoxidharze mit hoher Viskosität, beispielsweise mit einer Viskosität bei 20 °C oberhalb von 500 mPa·s, insbesondere oberhalb von 1'000 mPa·s, vermag das Amin der Formel (I) deren Viskosität erheblich zu senken.

Durch die Verwendung des Amins der Formel (I) als Verdünner sind niedrigviskose Härter für Epoxidharze zugänglich, welche ganz frei sind von nicht einbaubaren Verdünnern und so emissionsarme Epoxidharze mit hoher Härte, geringer Sprödigkeit und geringer Vergilbungsneigung ermöglichen.

Ein weiterer Gegenstand der Erfindung ist ein Härter, geeignet zum Aushärten von Epoxidharzen, umfassend das Amin der Formel (I) und mindestens ein Polyamin **A,** welches mindestens drei gegenüber Epoxidgruppen reaktive Aminwasserstoffe aufweist.

Geeignet als Polyamin **A** sind insbesondere die folgenden Polyamine:
- aliphatische, cycloaliphatische oder arylaliphatische primäre Diamine, insbesondere 2,2-Dimethyl-1,3-propandiamin, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- und 1,4-Diaminocyclohexan, Bis(4-aminocyclohexyl)methan (H₁₂-MDA), Bis(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (= Isophorondiamin oder IPDA), 2-und 4-Methyl-1,3-diaminocyclohexan und Mischungen davon, 1,3- und 1,4-Bis(aminomethyl)cyclohexan, 2,5(2,6)-Bis(aminomethyl)bicyclo[2.2.1]heptan (NBDA), 3(4), 8(9)-Bis(aminomethyl)tricyclo[5.2.1.0^{2,6}]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan sowie 1,3- und 1,4-Bis(aminomethyl)benzol;
- aliphatische, cycloaliphatische oder arylaliphatische primäre Triamine, insbesondere 4-Aminomethyl-1,8-octandiamin, 1,3,5-Tris(aminomethyl)benzol, 1,3,5-Tris(aminomethyl)cyclohexan, Tris(2-aminoethyl)amin, Tris(2-aminopropyl)amin und Tris(3-aminopropyl)amin;
- Ethergruppen-haltige aliphatische primäre Diamine, insbesondere Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin und höhere Oligomere dieser Diamine, Bis(3-aminopropyl)polytetrahydrofurane und andere Polytetrahydrofuran-diamine, cycloaliphatische Ethergruppen-haltige Diamine aus der Propoxylierung und nachfolgenden Aminierung von 1,4-Dimethylolcyclohexan, erhältlich insbesondere als Jeffamine^{®} RFD-270 (von Huntsman), sowie Polyoxyalkylendiamine, welche typischerweise Produkte aus der Aminierung von Polyoxyalkylendiolen darstellen und beispielsweise erhältlich sind unter dem Namen Jeffamine^{®} (von Huntsman), unter dem Namen Polyetheramine (von BASF) oder unter dem Namen PC Amine^{®} (von Nitroil). Insbesondere geeignete Polyoxyalkylendiamine sind Jeffamine^{®} D-230, Jeffamine^{®} D-400, Jeffamine^{®} D-2000, Jeffamine^{®} EDR-104, Jeffamine^{®} EDR-148 und Jeffamine^{®} EDR-176, sowie entsprechende Amine von BASF oder Nitroil;
- primäre Polyoxyalkylentriamine, welche typischerweise Produkte aus der Aminierung von Polyoxyalkylentriolen darstellen und beispielsweise erhältlich sind unter dem Namen Jeffamine^{®} (von Huntsman), unter dem Namen Polyetheramine (von BASF) oder unter dem Namen PC Amine^{®} (von Nitroil), insbesondere Jeffamine^{®} T-403, Jeffamine^{®} T-3000, Jeffamine^{®} T-5000, sowie entsprechende Amine von BASF oder Nitroil;
- tertiäre Aminogruppen aufweisende Polyamine mit zwei primären aliphatischen Aminogruppen, wie insbesondere N,N'-Bis(aminopropyl)-piperazin, N,N-Bis(3-aminopropyl)methylamin, N,N-Bis(3-aminopropyl)ethylamin, N,N-Bis(3-aminopropyl)propylamin, N,N-Bis(3-aminopropyl)cyclohexylamin, N,N-Bis(3-aminopropyl)-2-ethyl-hexylamin, sowie die Produkte aus der doppelten Cyanoethylierung und nachfolgender Reduktion von Fettaminen, welche abgeleitet sind von natürlichen Fettsäuren, wie N,N-Bis(3-aminopropyl)dodecylamin und N,N-Bis(3-aminopropyl)talgalkylamin, erhältlich als Triameen^{®} Y12D und Triameen^{®} YT (von Akzo Nobel);
- tertiäre Aminogruppen aufweisende Polyamine mit drei primären aliphatischen Aminogruppen, wie insbesondere Tris(2-aminoethyl)amin, Tris(2-aminopropyl)amin und Tris(3-aminopropyl)amin;
- sekundäre Aminogruppen aufweisende Polyamine mit zwei primären aliphatischen Aminogruppen, wie insbesondere 3-(2-Aminoethyl)aminopropylamin, Bis(hexamethylen)triamin (BHMT), Diethylentriamin (DETA), Triethylentetramin (TETA), Tetraethylenpentamin (TEPA), Pentaethylenhexamin (PEHA) und höhere Homologe linearer Polyethylenamine wie Polyethylenpolyamin mit 5 bis 7 Ethylenamin-Einheiten (sogenanntes "higher ethylenepolyamine", HEPA), Produkte aus der mehrfachen Cyanoethylierung oder Cyanobutylierung und anschliessender Hydrierung von primären Di- und Polyaminen mit mindestens zwei primären Aminogruppen, wie Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-aminopropyl)-1,4-diaminobutan, N5-(3-Aminopropyl)-2-methyl-1,5-pentandiamin, N3-(3-Aminopentyl)-1,3-pentandiamin, N5-(3-Amino-1-ethylpropyl)-2-methyl-1,5-pentandiamin und N,N'-Bis(3-amino-1-ethylpropyl)-2-methyl-1,5-pentandiamin;
- eine primäre und eine sekundäre Aminogruppe aufweisende Polyamine, wie insbesondere N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, N-(2-Aminoethyl)piperazin, N-Methyl-1,3-propandiamin, N-Butyl-1,3-propandiamin, N-(2-Ethylhexyl)-1,3-propandiamin, N-Cyclohexyl-1,3-propandiamin, 3-Methylamino-1-pentylamin, 3-Ethylamino-1-pentylamin, 3-Cyclohexylamino-1-pentylaminFettdiamine wie N-Cocoalkyl-1,3-propandiamin und Produkte aus der Michael-artigen Additionsreaktion von primären aliphatischen Diaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1, weiterhin Produkte aus der partiellen reduktiven Alkylierung von primären aliphatischen Polyaminen mit Aldehyden oder Ketonen, insbesondere N-2-Ethylhexyl-1,3-bis(aminomethyl)benzol, sowie partiell styrolisierte Polyamine wie Gaskamine^{®} 240 (von Mitsubishi Gas Chemical (MGC));
- aromatische Polyamine, wie insbesondere m- und p-Phenylendiamin, 4,4'-, 2,4' und 2,2'-Diaminodiphenylmethan, 3,3'-Dichloro-4,4'-diaminodiphenylmethan (MOCA), 2,4- und 2,6-Toluylendiamin, Mischungen von 3,5-Dimethylthio-2,4- und -2,6-toluylendiamin (erhältlich als Ethacure^{®} 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)benzolsulfonamid, 5,5'-Methylendianthranilsäure, Dimethyl-(5,5'-methylendianthranilat), 1,3-Propylen-bis(4-aminobenzoat), 1,4-Butylen-bis(4-aminobenzoat), Polytetramethylenoxid-bis(4-aminobenzoat) (erhältlich als Versalink^{®} von Air Products), 1,2-Bis(2-aminophenyl-thio)ethan, 2-Methylpropyl-(4-chloro-3,5-diaminobenzoat) und tert.Butyl-(4-chloro-3,5-diaminobenzoat);
- Addukte der genannten Polyamine mit Epoxiden und Epoxidharzen, insbesondere Addukte mit Diepoxiden im Molverhältnis von ungefähr 2/1, Addukte mit Monoepoxiden im Molverhältnis von mindestens 1/1, sowie Umsetzungsprodukte aus Aminen und Epichlorhydrin, insbesondere jenes von 1,3-Bis-(aminomethyl)benzol, kommerziell erhältlich als Gaskamine^{®} 328 (von MGC);
- Polyamidoamine, welche Umsetzungsprodukte aus einer ein- oder mehrwertigen Carbonsäure, beziehungsweise deren Ester oder Anhydride, insbesondere einer Dimerfettsäure, und einem im stöchiometrischen Überschuss eingesetzten aliphatischen, cycloaliphatischen oder aromatischen Polyamin, insbesondere einem Polyalkylenamin wie beispielsweise DETA oder TETA, darstellen, insbesondere die kommerziell erhältlichen Polyamidoamine Versamid^{®} 100, 125, 140 und 150 (von Cognis), Aradur^{®} 223, 250 und 848 (von Huntsman), Euretek^{®} 3607 und 530 (von Huntsman) und Beckopox^{®} EH 651, EH 654, EH 655, EH 661 und EH 663 (von Cytec); und
- Phenalkamine, auch Mannich-Basen genannt, welche Umsetzungsprodukte einer Mannich-Reaktion von Phenolen, insbesondere Cardanol, mit Aldehyden, insbesondere Formaldehyd, und Polyaminen darstellen, insbesondere die kommerziell erhältlichen Phenalkamine Cardolite^{®} NC-541, NC-557, NC-558, NC-566, Lite 2001 und Lite 2002 (von Cardolite), Aradur^{®} 3440, 3441, 3442 und 3460 (von Huntsman) und Beckopox^{®} EH 614, EH 621, EH 624, EH 628 und EH 629 (von Cytec).

Bevorzugt ist das Polyamin **A** ausgewählt aus der Gruppe bestehend aus 1,5-Diamino-2-methylpentan (MPMD), 2-Butyl-2-ethyl-1,5-pentandiamin (C11-Neodiamin), 1,6-Hexandiamin, 2,2,4- und 2,4,4-Trimethylhexamethylendiamin (TMD), 1,12-Dodecandiamin, 1,3-Diaminocyclohexan, Bis(4-aminocyclohexyl)-methan (HMDA), Bis(4-amino-3-methylcyclohexyl)methan, 1-Amino-3-amino-methyl-3,5,5-trimethylcyclohexan (IPD), 1,3-Bis(aminomethyl)cyclohexan, 1,3-Bis(aminomethyl)benzol (MXDA), Bishexamethylentriamin (BHMT), Diethylentriamin (DETA), Triethylentetramin (TETA), Tetraethylenpentamin (TEPA), Pentaethylenhexamin (PEHA) und höhere Homologe linearer Polyethylenamine wie Polyethylenpolyamin mit 5 bis 7 Ethylenamin-Einheiten (HEPA), Dipropylentriamin (DPTA), N-(2-Aminoethyl)-1,3-propandiamin (N3-Amin), N,N'-Bis(3-aminopropyl)ethylendiamin (N4-Amin), cycloaliphatischen ethergruppenhaltigen Diaminen aus der Propoxylierung und nachfolgender Aminierung von 1,4-Dimethylolcyclohexan mit einem Molekulargewicht im Bereich von 200 bis 300 g/mol, insbesondere Jeffamine^{®} RFD-270 (von Huntsman), Polyoxyalkylendiaminen und Polyoxyalkylentriaminen mit einem Molekulargewicht im Bereich von 200 bis 500 g/mol, insbesondere die Typen Jeffamine^{®} D-230, Jeffamine^{®} D-400 und Jeffamine^{®} T-403, sowie Derivaten dieser Polyamine in Form von Addukten mit Epoxiden, insbesondere Monoepoxiden oder Epoxidharzen, oder in Form von Michael-Addukten, Mannich-Basen oder Polyamidoaminen.

Diese bevorzugten Polyamine **A** sind besonders gut verträglich mit Epoxidharzen und ermöglichen Filme von hoher Qualität.

Besonders bevorzugt stellt das Polyamin **A** ein Addukt mit einem Epoxid dar, insbesondere ein Addukt mit einem Epoxidharz oder mit einem Monoepoxid. Solche Addukte zeigen ausgezeichnete Eigenschaften als Härter für Epoxidharze, insbesondere eine schnelle Aushärtungsgeschwindigkeit, auch bei tiefen Temperaturen, und eine wenig ausgeprägte Neigung zu Blushing-Effekten. Sie ergeben Filme von ausgezeichneter Qualität, sind aber aufgrund ihrer hohen Viskosität für Beschichtungsanwendungen nur geeignet, wenn sie stark verdünnt werden. Dies wird im Stand der Technik typischerweise durch Mischungen enthaltend nicht einbaubare Verdünner und grössere Mengen an kleinen, relativ flüchtigen primären Diaminen erreicht. Soll dabei aber auf nicht einbaubare Verdünner verzichtet werden, so sind solche Härter entweder zu hochviskos oder führen zu starken Blushing-Effekten. Durch das Verdünnen solcher Addukte mit dem Amin der Formel (I) sind Härter für Epoxidharze mit hervorragenden Eigenschaften für emissionsarme Beschichtungen zugänglich.

Besonders bevorzugt stellt das Polyamin **A** ein Addukt mit einem aromatischen Monoepoxid dar. Solche Addukte weisen eine moderate Viskosität auf und sind gut verträglich mit Epoxidharzen.
Besonders bevorzugte Monoepoxide sind aromatische Glycidylether, insbesondere die Kresylglycidylether. Als Kresylglycidylether geeignet sind alle isomeren Kresylglycidylether und Gemische davon, insbesondere kommerziell erhältliche Typen wie insbesondere Araldite^{®} DY-K (von Huntsman), Polypox™ R6 (von Dow), Heloxy™ KR (von Hexion) oder Erisys^{®} GE-10 (von CVC Spec. Chem.). Ein solches Addukt weist eine besonders gute Verträglichkeit mit den üblichen Epoxidharz-Produkten auf und ermöglicht ausgehärtete Filme von hohem Glanz und hoher Härte.

In einem Aspekt der Erfindung enthält der Härter mindestens zwei verschiedene Polyamine **A,** insbesondere mindestens ein Polyamin **A1,** welches bevorzugt ein Addukt mit einem Epoxid darstellt, und mindestens ein Polyamin **A2,** welches ein nicht-adduktiertes Polyamin darstellt. Durch die Verdünnung einer solchen Mischung aus Polyamin **A1** und Polyamin **A2** mit dem Amin der Formel (I) kann sowohl die Viskosität als auch der Gehalt an primären Aminogruppen so tief gehalten werden, dass emissionsarme Beschichtungen mit guter Verarbeitbarkeit, geringer Neigung zu Blushing-Effekten, schneller Aushärtung und geringer Sprödigkeit erhältlich sind.

Bevorzugt liegt das Amin der Formel (I) im Härter in einer solchen Menge vor, dass seine Aminwasserstoffe 1 bis 75 %, bevorzugt 2 bis 50 %, insbesondere 5 bis 30 %, der gesamthaft im Härter vorhandenen Aminwasserstoffe ausmachen.

Weiterhin bevorzugt liegt das Amin der Formel (I) im Härter in einer solchen Menge vor, dass sein Gewichtsanteil 1 bis 95 %, bevorzugt 5 bis 75 %, insbesondere 5 bis 50 %, der Summe aller gegenüber Epoxidgruppen reaktiven Amine ausmacht.
Solche Härter zeichnen sich durch eine niedrige Viskosität aus und ermöglichen Epoxidharz-Beschichtungen mit hoher Aushärtungsgeschwindigkeit, kaum Neigung zu Blushing-Effekten und hoher Härte bei geringer Sprödigkeit.

Der Härter kann weiterhin mindestens einen Beschleuniger enthalten. Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; weiterhin tertiäre Amine wie insbesondere 1,4-Diazabicyclo[2.2.2]octan, Benzyldimethylamin, α-Methylbenzyldimethylamin, Triethanolamin, Dimethyl-aminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo[5.4.0]undec-7-en, Guanidine wie insbesondere 1,1,3,3-Tetramethylguanidin, Phenole, insbesondere Bisphenole, Phenol-Harze und Mannich-Basen wie insbesondere 2-(Dimethylaminomethyl)phenol, 2,4,6-Tris(dimethylaminomethyl)phenol und Polymere aus Phenol, Formaldehyd und N,N-Dimethyl-1,3-propandiamin, Phosphite wie insbesondere Di- und Triphenylphosphite, sowie Mercaptogruppen aufweisende Verbindungen.
Bevorzugte Beschleuniger sind Salicylsäure und 2,4,6-Tris(dimethylaminomethyl)phenol.

Der Härter kann weiterhin mindestens einen nicht einbaubaren Verdünner enthalten, insbesondere Xylol, 2-Methoxyethanol, Dimethoxyethanol, 2-Ethoxyethanol, 2-Propoxyethanol, 2-lsopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykol-monomethylether, Diethylenglykol-monoethylether, Diethylenglykol-mono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, N-Methylpyrrolidon, Diphenylmethan, Diisopropylnaphthalin, Erdölfraktionen wie zum Beispiel Solvesso^{®}-Typen (von Exxon), Alkylphenole wie tert.Butylphenol, Nonylphenol, Dodecylphenol und 8,11,14-Pentadecatrienylphenol (Cardanol, aus Cashewschalen-Öl, erhältlich beispielsweise als Cardolite NC-700 von Cardolite Corp., USA), styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- und Sulfonsäureester und Sulfonamide. Bevorzugt sind Benzylalkohol, Dodecylphenol, tert.Butylphenol, styrolisiertes Phenol, ethoxyliertes Phenol und phenolgruppenhaltige aromatische Kohlenwasserstoffharze, insbesondere die Novares^{®}-Typen LS 500, LX 200, LA 300 und LA 700 (von Rütgers).

Bevorzugt enthält der Härter keinen oder nur einen geringen Gehalt an nicht einbaubaren Verdünnern, besonders bevorzugt weniger als 25 Gewichts-%, insbesondere weniger als 10 Gewichts-% und am meisten bevorzugt weniger als 5 Gewichts-%. Insbesondere werden dem Härter keine nicht einbaubaren Verdünner zugesetzt.

Der Härter kann weitere gegenüber Epoxidgruppen reaktive Substanzen enthalten, beispielsweise Monoamine, wie Hexylamin und Benzylamin; sekundäre aliphatische Polyamine; Mercaptogruppen aufweisende Verbindungen, insbesondere die Folgenden:
- flüssige Mercaptan-terminierte Polysulfid-Polymere, bekannt unter dem Markennamen Thiokol^{®} (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 und LP-2; sowie weiterhin bekannt unter dem Markennamen Thioplast^{®} (von Akzo Nobel), insbesondere die Typen G 10, G 112, G 131, G 1, G 12, G 21, G 22, G 44 und G 4;
- Mercaptan-terminierte Polyoxyalkylen-Ether, erhältlich beispielsweise durch Umsetzung von Polyoxyalkylendi- und -triolen entweder mit Epichlorhydrin oder mit einem Alkylenoxid, gefolgt von Natriumhydrogensulfid;
- Mercaptan-terminierte Verbindungen in Form von Polyoxyalkylen-Derivaten, bekannt unter dem Markennamen Capcure^{®} (von Cognis), insbesondere die Typen WR-8, LOF und 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri-(3-mercaptopropionat) und Glykoldi-(3-mercaptopropionat), sowie die Veresterungsprodukte von Polyoxyalkylendiolen und -triolen, ethoxyliertem Trimethylolpropan und Polyester-Diolen mit Thiocarbonsäuren wie Thioglykolsäure und 2- oder 3-Mercaptopropionsäure; und
- weitere Mercaptogruppen aufweisende Verbindungen, wie insbesondere 2,4,6-Trimercapto-1,3,5-triazin, 2,2'-(Ethylendioxy)-diethanthiol (Triethylenglykol-dimercaptan) und Ethandithiol.

Ein weiterer Gegenstand der Erfindung ist eine Epoxidharz-Zusammensetzung, enthaltend mindestens ein Epoxidharz und den vorgängig beschriebenen Härter.

Als Epoxidharz sind übliche technische Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.
Als Epoxidharz besonders geeignet sind sogenannte Polyepoxid-Flüssigharze, im folgenden als "Flüssigharz" bezeichnet. Diese weisen eine Glasübergangstemperatur unterhalb von 25°C auf.

Ebenfalls möglich als Epoxidharz sind sogenannte Festharze, welche eine Glasübergangstemperatur oberhalb von 25°C aufweisen und sich zu bei 25°C schüttfähigen Pulvern zerkleinern lassen.

Geeignete Epoxidharze sind insbesondere aromatische Epoxidharze, insbesondere die Glycidylisierungsprodukte von:
- Bisphenol-A, Bisphenol-F oder Bisphenol-A/F, wobei A für Aceton und F für Formaldehyd steht, welche als Edukte zur Herstellung dieser Bisphenole dienten. Im Fall von Bisphenol-F können auch Stellungsisomere vorhanden sein, insbesondere abgeleitet von 2,4'- und 2,2'-Hydroxyphenylmethan;
- Dihydroxybenzol-Derivaten wie Resorcin, Hydrochinon und Brenzkatechin;
- weiteren Bisphenolen oder Polyphenolen wie Bis(4-hydroxy-3-methylphenyl)methan, 2,2-Bis(4-hydroxy-3-methylyphenyl)propan (Bisphenol-C), Bis-(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)heptan, 2,4-Bis(4-hydroxyphenyl)-2-methylbutan, 2,4-Bis-(3,5-dimethyl-4-hydroxyphenyl)-2-methylbutan, 1,1-Bis(4-hydroxyphenyl)-cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxynaphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether und Bis-(4-hydroxyphenyl)sulfon;
- Kondensationsprodukten von Phenolen mit Formaldehyd, die unter sauren Bedingungen erhalten werden, wie Phenol-Novolaken oder Kresol-Novolaken, auch Bisphenol-F-Novolake genannt;
- aromatischen Aminen, wie Anilin, Toluidin, 4-Aminophenol, 4,4'-Methylendi-phenyldiamin, 4,4'-Methylendiphenyldi-(N-methyl)amin, 4,4'-[1,4-Phenylen-bis(1-methylethyliden)]bisanilin (Bisanilin-P), 4,4'-[1,3-Phenylen-bis(1-me-thylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂-bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromo-neopentylglykol, Rizinusöl, Trimethylolpropan, Trimethylolethan, Pentaerythrol, Sorbit oder Glycerin, sowie alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat und Triglycidylisocyanurat, sowie Umsetzungsprodukte von Epichlorhydrin und Hydantoin.
- Epoxidharze aus der Oxidation von Olefinen, wie insbesondere Vinylcylohexen, Dicyclopentadien, Cyclohexadien, Cyclododecadien, Cyclododecatrien, Isopren, 1,5-Hexadien, Butadien, Polybutadien oder Divinylbenzol.

Bevorzugte ist das Epoxidharz ein Flüssigharz auf der Basis eines Bisphenols, insbesondere ein Diglycidylether von Bisphenol-A, Bisphenol-F oder Bisphenol-A/F, wie sie kommerziell beispielsweise von Dow, Huntsman und Hexion erhältlich sind. Diese Flüssigharze weisen eine für Epoxidharze niedrige Viskosität und im ausgehärteten Zustand gute Eigenschaften als Beschichtung auf. Sie können gegebenenfalls in Kombination mit Bisphenol A-Festharz oder Bisphenol-F-Novolak-Epoxidharz vorhanden sein.

Das Epoxidharz kann einen Reaktivverdünner, insbesondere einen mindestens eine Epoxidgruppe aufweisenden Reaktivverdünner, enthalten. Als Reaktivverdünner geeignet sind beispielsweise die Glycidylether von ein- oder mehrwertigen Phenolen und aliphatischen oder cycloaliphatischen Alkoholen, wie insbesondere die bereits genannten Polyglycidylether von Di- oder Polyolen, sowie weiterhin insbesondere Phenylglycidylether, Kresylglycidylether, Benzylglycidylether, p-n-Butyl-phenylglycidylether, p-tert.Butyl-phenylglycidylether, Nonylphenylglycidylether, Allylglycidylether, Butylglycidylether, Hexylglycidylether, 2-Ethylhexylglycidylether, sowie Glycidylether von natürlichen Alkoholen, wie zum Beispiel C₈- bis C₁₀-Alkylglycidylether oder C₁₂- bis C₁₄-Alkylglycidylether. Die Zugabe eines Reaktivverdünners zum Epoxidharz bewirkt eine Reduktion der Viskosität, sowie eine Reduktion der Glasübergangstemperatur und der mechanischen Werte.

Gegebenenfalls enthält die Epoxidharz-Zusammensetzung weitere Bestandteile, insbesondere in Epoxidharz-Zusammensetzungen üblicherweise eingesetzte Hilfs- und Zusatzstoffe, beispielsweise die Folgenden:
- Lösemittel, Verdünner, Filmbildehilfsmittel oder Extender, wie insbesondere die bereits genannten nicht einbaubaren Verdünner;
- Reaktivverdünner, insbesondere Epoxidgruppen aufweisende Reaktivverdünner, wie sie vorgängig erwähnt wurden, epoxidiertes Sojaöl oder Leinöl, Acetoacetatgruppen aufweisende Verbindungen, insbesondere acetoacetylierte Polyole, Butyrolakton, Carbonate, Aldehyde, sowie weiterhin Isocyanate und Reaktivgruppen-aufweisende Silikone;
- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere chlorsulfonierte Polyethylene und Fluor-haltige Polymere, Sulfonamid-modifizierte Melamine und gereinigte Montan-Wachse;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Eisenglimmer, Dolomite, Wollastonite, Kaoline, Mica (Kalium-Aluminium-Silikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Zink, Silber oder Stahl, PVC-Pulver oder Hohlkugeln;

- Fasern, insbesondere Glasfasern, Kohlefasern, Metallfasern, Keramikfasern oder Kunststofffasern wie Polyamidfasern oder Polyethylenfasern;
- Pigmente, insbesondere Titandioxid und Eisenoxide;
- die vorgenannten Beschleuniger;
- Rheologie-Modifizierer, insbesondere Verdicker oder Antiabsetzmittel;
- Haftverbesserer, insbesondere Organoalkoxysilane;
- Stabilisatoren gegen Oxidation, Wärme, Licht und UV-Strahlung;
- flammhemmende Substanzen, insbesondere Aluminiumhydroxid (ATH), Magnesiumdihydroxid (MDH), Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinol-diphosphat-Oligomer, Tetraphenylresorcinoldiphosphit, Ethylendiamindiphosphat und Bisphenol-A-bis(diphenylphosphat), Tris(chloroethyl)phosphat, Tris(chloropropyl)phosphat und Tris(dichloroisopropyl)phosphat, Tris[3-bromo-2,2-bis(bromomethyl)propyl]phosphat, Tetrabromo-Bisphenol-A, Bis(2,3-dibromopropylether) von Bisphenol A, bromierte Epoxidharze, Ethylen-bis-(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan und Chlorparaffine;
- oberflächenaktive Substanzen, insbesondere Netzmittel, Verlaufsmittel, Entlüftungsmittel und Entschäumer;
- Biozide, wie beispielsweise Algizide, Fungizide oder das Pilzwachstum hemmende Substanzen.

Bevorzugt enthält die Epoxidharz-Zusammensetzung weitere Hilfs- und Zusatzstoffe, insbesondere Netzmittel, Verlaufsmittel, Entschäumer, Stabilisatoren, Pigmente und Katalysatoren, insbesondere Salicylsäure oder 2,4,6-Tris(dimethylaminomethyl)phenol.

Bevorzugt enthält die Epoxidharz-Zusammensetzung keinen oder nur einen geringen Gehalt an nicht einbaubaren Verdünnern, besonders bevorzugt weniger als 10 Gewichts-%, insbesondere weniger als 5 Gewichts-%, am meisten bevorzugt weniger als 2 Gewichts-%.

In der Epoxidharz-Zusammensetzung liegt das Verhältnis der Anzahl von gegenüber Epoxidgruppen reaktiven Gruppen gegenüber der Anzahl Epoxidgruppen bevorzugt im Bereich von 0.5 bis 1.5, insbesondere 0.7 bis 1.2.

Die in der Epoxidharz-Zusammensetzung vorhandenen Aminwasserstoffe und gegebenenfalls vorhandene weitere gegenüber Epoxidgruppen reaktive Gruppen reagieren mit den Epoxidgruppen unter deren Ringöffnung (Additionsreaktion). Als Ergebnis dieser Reaktionen polymerisiert die Zusammensetzung und härtet schliesslich aus. Dem Fachmann ist bekannt, dass primäre Aminogruppen gegenüber Epoxidgruppen difunktionell sind und eine primäre Aminogruppe somit als zwei gegenüber Epoxidgruppen reaktive Gruppen zählt.

Insbesondere ist die Epoxidharz-Zusammensetzung eine zweikomponentige Zusammensetzung, bestehend aus
(i) einer Harz-Komponente enthaltend mindestens ein Epoxidharz und
(ii) einer Härter-Komponente enthaltend den beschriebenen Härter.

Die Komponenten der zweikomponentigen Zusammensetzung werden jeweils in einem eigenen Gebinde gelagert. Weitere Bestandteile der zweikomponentigen Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern. Zur Anwendung der zweikomponentigen Epoxidharz-Zusammensetzung werden die Harz- und die Härter-Komponente kurz vor oder während der Applikation miteinander vermischt. Das Mischungsverhältnis zwischen den beiden Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1:10 bis 10:1.

Die Vermischung der beiden Komponenten erfolgt mittels eines geeigneten Verfahrens; sie kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung erfolgt insbesondere bei Umgebungstemperatur, welche typischerweise im Bereich von etwa 5 bis 50°C, bevorzugt bei etwa 10 bis 30°C, liegt.

Mit der Vermischung der beiden Komponenten beginnt die Aushärtung durch chemische Reaktion, wie vorgängig beschrieben. Die Aushärtung erfolgt insbesondere bei Umgebungstemperatur. Sie erstreckt sich typischerweise über einige Tage bis Wochen, bis sie unter den gegebenen Bedingungen weitgehend abgeschlossen ist. Die Dauer hängt unter anderem von der Temperatur, der Reaktivität der Bestandteile und deren Stöchiometrie sowie der Gegenwart von Beschleunigern ab.

Ein weiterer Gegenstand der Erfindung ist somit auch eine ausgehärtete Zusammensetzung erhalten aus der Aushärtung einer Epoxidharz-Zusammensetzung wie im vorliegenden Dokument beschrieben.

Die Applikation der Epoxidharz-Zusammensetzung erfolgt auf mindestens ein Substrat, wobei die Folgenden besonders geeignet sind:
- Glas, Glaskeramik, Beton, Mörtel, Backstein, Ziegel, Gips und Natursteine wie Granit oder Marmor;
- Metalle und Legierungen, wie Aluminium, Eisen, Stahl und Buntmetalle, inklusive oberflächenveredelte Metalle und Legierungen, wie verzinkte und verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe und weitere sogenannte Polymer-Composites;
- Kunststoffe, insbesondere Hart- und Weich-PVC, ABS, Polycarbonat (PC), Polyamid (PA), Polyester, PMMA, Epoxidharze, PUR, POM, PO, PE, PP, EPM und EPDM, wobei die Kunststoffe gegebenenfalls mittels Plasma, Corona oder Flammen oberflächenbehandelt sind;
- Faserverstärkte Kunststoffe, wie Kohlefaser-verstärkte Kunststoffe (CFK), Glasfaser-verstärkte Kunststoffe (GFK) und Sheet Moulding Compounds (SMC);
- beschichtete Substrate, wie pulverbeschichtete Metalle oder Legierungen;
- Farben und Lacke.

Die Substrate können bei Bedarf vor dem Applizieren der Epoxidharz-Zusammensetzung vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten und/oder Abblasen, sowie weiterhin Behandeln mit Reinigern oder Lösemitteln oder das Aufbringen eines Haftvermittlers, einer Haftvermittlerlösung oder eines Primers.

Die beschriebene Epoxidharz-Zusammensetzung ist vorteilhaft verwendbar als Faserverbundwerkstoff (Composite), Vergussmasse, Dichtstoff, Klebstoff, Belag, Beschichtung, Anstrich, Lack, Versiegelung, Grundierung oder Primer. Insbesondere verwendbar ist sie als Vergussmasse, Dichtstoff und Klebstoff, wie beispielsweise als Elektrovergussmasse, Abdichtungsmasse, Karrosserieklebstoff, Sandwichelementklebstoff, Halbschalenklebstoff, beispielsweise für Rotorblätter von Windkraftanlagen, Brückenelementklebstoff oder Verankerungsklebstoff; sowie weiterhin als Belag, Beschichtung, Anstrich, Lack, Versiegelung, Grundierung und Primer für Bau- und Industrieanwendungen, wie insbesondere als Bodenbelag und Bodenbeschichtung für Innenräume wie Büros, Industriehallen, Turnhallen oder Kühlräume, oder im Aussenbereich für Balkone, Terrassen, Parkdecks, Brücken oder Dächer, als Schutzbeschichtung für Beton, Zement, Metalle, Kunststoffe oder Holz, beispielsweise zur Oberflächenversiegelung von Holzkonstruktionen, Fahrzeugen, Ladeflächen, Tanks, Silos, Schächten, Rohrleitungen, Pipelines, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden; sowie weiterhin als Voranstrich, Haftanstrich, Korrosionsschutz-Primer oder zur Hydrophobierung von Oberflächen. Auf die vollständig oder teilweise ausgehärtete Epoxidharz-Zusammensetzung kann insbesondere bei ihrer Verwendung als Beschichtung, Belag oder Anstrich eine weitere Beschichtung, ein weiterer Belag, oder ein weiterer Anstrich appliziert werden, wobei es sich bei dieser weiteren Schicht ebenfalls um eine Epoxidharz-Zusammensetzung handeln kann, aber auch um ein anderes Material, insbesondere um eine Polyurethan- oder Polyharnstoff-Beschichtung.

Besonders vorteilhaft verwendbar ist die beschriebene Epoxidharz-Zusammensetzung als Beschichtung. Als Beschichtung werden dabei flächig aufgebrachte Beläge aller Art verstanden, insbesondere auch Anstriche, Lacke, Versiegelungen, Grundierungen und Primer, wie vorgängig beschrieben. Insbesondere vorteilhaft verwendbar ist die beschriebene Epoxidharz-Zusammensetzung in emissionsarmen Epoxidharz-Produkten mit Öko-Gütesiegeln, beispielsweise nach Emicode (EC1 Plus), AgBB, DIBt, Der Blaue Engel, AFSSET, RTS (M1) und US Green Building Council (LEED).

Als Beschichtung wird die Epoxidharz-Zusammensetzung vorteilhaft in einem Verfahren zum Beschichten verwendet, wobei sie eine flüssige Konsistenz mit niedriger Viskosität und guten Verlaufseigenschaften aufweist und insbesondere als selbstverlaufende Beschichtung auf überwiegend ebene Flächen oder als Anstrich appliziert werden kann. Bevorzugt weist die Epoxidharz-Zusammensetzung bei dieser Applikation unmittelbar nach dem Vermischen der Harz- und der Härter-Komponente eine Viskosität, gemessen bei 20°C, im Bereich von 300 bis 2'000 mPa·s, bevorzugt im Bereich von 300 bis 1'500 mPa·s, besonders bevorzugt im Bereich von 300 bis 1'200 mPa·s, auf. Die vermischte Zusammensetzung wird innerhalb der Verarbeitungszeit flächig als dünner Film mit einer Schichtdicke von typischerweise etwa 50 µm bis etwa 5 mm auf ein Substrat appliziert, typischerweise bei Umgebungstemperatur. Die Applikation erfolgt insbesondere durch Aufgiessen auf das zu beschichtende Substrat und anschliessendem gleichmässigem Verteilen mit Hilfe beispielsweise eines Rakels oder einer Zahntraufel. Die Applikation kann aber auch mit einem Pinsel oder Roller oder als Spritzapplikation erfolgen, beispielsweise als Korrosionsschutzbeschichtung auf Stahl.

Bei der Aushärtung entstehen typischerweise weitgehend klare, glänzende und nichtklebrige Filme von hoher Härte, geringer Sprödigkeit und einer geringen Neigung zu Vergilbung, welche eine gute Haftung zu verschiedensten Substraten aufweisen.

Eine Film von hoher Härte und geringer Sprödigkeit weist bevorzugt eine Königshärte (Pendelhärte nach König, gemessen nach DIN EN ISO 1522) im Bereich von 100 bis 200 s, insbesondere 120 bis 180 s, auf. Eine noch höhere Königshärte weist typischerweise auch eine erhöhte Sprödigkeit auf, und eine geringere Königshärte ist zu weich für viele Beschichtungsanwendungen.

Ein weiterer Gegenstand der Erfindung ist ein Artikel enthaltend eine ausgehärtete Zusammensetzung, erhalten durch die Aushärtung der beschriebenen Epoxidharz-Zusammensetzung. Die ausgehärtete Zusammensetzung liegt dabei insbesondere in Form einer Beschichtung vor.

Die beschriebene Epoxidharz-Zusammensetzung zeichnet sich durch vorteilhafte Eigenschaften aus. Sie ist niedrigviskos und härtet auch bei feuchtkalten Bedingungen schnell und weitgehend ohne Blushing-Effekte aus, wobei klare Filme von hoher Härte, geringer Sprödigkeit und geringer Vergilbungsneigung erhältlich sind, sogar mit geringen Anteilen oder ganz ohne die Verwendung von nicht einbaubaren Verdünnern und mit geringen Anteilen oder ganz ohne die Verwendung von kleinen, relativ flüchtigen primären Diaminen. Mit der beschriebenen Epoxidharz-Zusammensetzung sind emissionsarme Epoxidharz-Produkte zugänglich, welche die Bedingungen für viele Öko-Gütesiegel erfüllen und gleichzeitig hohen Ansprüchen bezüglich Arbeitssicherheit, Verarbeitungs- und Gebrauchseigenschaften genügen.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.
"AHEW" steht für das Aminwasserstoff-Equivalentgewicht.
"EEW" steht für das Epoxid-Equivalentgewicht.
"MGC" steht für "Mitsubishi Gas Chemical".
"GT" steht für "Gewichtsteile".

### 1. Beschreibung der Messmethoden

Der **Amingehalt,** das heisst der totale Gehalt an Aminogruppen in den hergestellten Verbindungen, wurde titrimetrisch bestimmt (mit 0.1N HClO₄ in Eisessig, gegen Kristallviolett) und ist stets angegeben in mmol N/g. **Infrarotspektren** (FT-IR) wurden als unverdünnte Filme auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹).

**GC/MS** wurde unter folgenden Bedingungen durchgeführt: Säule Agilent VF-5ms, 30 m×0.25 mm, 0.25 µm Filmdicke; Aufheizrate 15 °C/min von 60 °C auf 320 °C, dann 15 min. bei 320 °C gehalten; Trägergas He mit konstanter Flussrate von 1.1 ml/min; Injektor Split 25:1, Temperatur 230 °C; Ionisationsmethode Cl⁺ (Methanol).

Die **Viskosität** wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10-100 s⁻¹) gemessen.

### 2. verwendete Substanzen:

- EP-Addukt 1:: Umsetzungsprodukt aus 116.0 GT 1,5-Diamino-2-methylpentan und 182 GT Araldite^{®} DY-K; AHEW = 99.4 g/Eq; Viskosität (20°C) = 5'800 mPa·s
- EP-Addukt 2:: Umsetzungsprodukt aus 136.2 GT 1,3-Bis(aminomethyl)-benzol und 182 GT Araldite^{®} DY-K; AHEW = 106.1 g/Eq; Viskosität (20°C) = 28'100 mPa·s
- Aradur^{®} 3442: Phenalkamin; AHEW = 125 g/Eq;
- (Huntsman): Viskosität (20°C) = 10'210 mPa·s
- Gaskamine^{®} 240: Styrolisiertes 1,3-Bis(aminomethyl)benzol;
- (MGC): AHEW = 103 g/Eq; Viskosität (20°C) = 165 mPa·s
- Jeffamine^{®} RFD-: Cycloaliphatisches ethergruppenhaltiges Diamin aus der
- 270 (Huntsman): Propoxylierung und nachfolgender Aminierung von 1,4-Dimethylolcyclohexan; AHEW = 67 g/Eq
- Araldite^{®} DY-K: Monoglycidylether von Kresol;
- (Huntsman): EEW ca. 182 g/Eq
- Araldite^{®} GY 250: Bisphenol-A-Diglycidylether;
- (Huntsman): EEW ca. 187.5 g/Eq
- Araldite^{®} DY-E: Monoglycidylether eines C₁₂- bis C₁₄-Alkohols;
- (Huntsman): EEW ca. 290 g/Eq
- Ancamine^{®} K 54: 2,4,6-Tris(dimethylaminomethyl)phenol (Air Products)

### 3. Herstellung von Aminen

### Amin-1: 1,3-Bis(2-ethylhexylaminomethyl)benzol

In einem Rundkolben wurden 25.6 g (0.20 mol) 2-Ethylhexanal und 13.6 g (0.10 mol) 1,3-Bis(aminomethyl)benzol (von MGC) unter Stickstoffatmosphäre in ausreichend Isopropanol gelöst. Die Lösung wurde während 30 Minuten bei Raumtemperatur gerührt und anschliessend bei einem Wasserstoff-Druck von 80 bar, einer Temperatur von 80 °C und einem Fluss von 3 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die Lösung im Vakuum bei 80 °C eingeengt. Erhalten wurde eine klare, leicht gelbliche Flüssigkeit mit einer Viskosität von 140 mPa·s bei 20 °C, einem Amingehalt von 5.50 mmol N/g, einer Reinheit von 87.7 % (bestimmt mittels Gas-Chromatographie) und einem theoretischen AHEW von ca. 180.3 g/Eq.

FT-IR: 2956, 2923, 2857, 2811, 1457, 1378, 1156, 1113, 776, 726, 699. GC/MS: t_{R} = 15.56 min; m/z = 361.0 ([MH⁺]; theoretische Masse für C₂₄H₄₄N₂: 360.35).

### Amin-2: Reaktionsgemisch enthaltend 1,3-Bis(2-ethylhexylaminomethyl)benzol und N-2-Ethylhexyl-1,3-bis(aminomethyl)benzol

Auf die gleiche Weise wie für das Amin-1 beschrieben wurden 20.5 g (0.16 mol) 2-Ethylhexanal und 13.6 g (0.10 mol) 1,3-Bis(aminomethyl)benzol umgesetzt. Erhalten wurde eine klare, leicht gelbliche Flüssigkeit mit einer Viskosität von 100 mPa·s bei 20 °C, einem Amingehalt von 6.39 mmol N/g, einem Gehalt an 1,3-Bis(2-ethylhexylaminomethyl)benzol von 66.4 Gewichts-%, einem Gehalt an N-2-Ethylhexyl-1,3-bis(aminomethyl)benzol von 30.1 Gewichts-% (bestimmt mittels Gas-Chromatographie) und einem theoretischen AHEW von ca. 130.2 g/Eq.

### Amin-3 (Referenz): 1,3-Bis(benzylaminomethyl)benzol

Auf die gleiche Weise wie für das Amin-1 beschrieben wurden 21.2 g (0.20 mol) Benzaldehyd und 13.6 g (0.10 mol) 1,3-Bis(aminomethyl)benzol umgesetzt. Erhalten wurde eine klare, leicht gelbliche Flüssigkeit mit einer Viskosität von 230 mPa·s bei 20 °C, einem Amingehalt von 6.41 mmol N/g und einem theoretischen AHEW von ca. 158.2 g/Eq.

**Amin-4 (Referenz):** 1,3-Bis(2-methylpropylaminomethyl)benzol Auf die gleiche Weise wie für das Amin-1 beschrieben wurden 14.4 g (0.2 mol) Isobutyraldehyd und 13.6 g (0.1 mol) 1,3-Bis(aminomethyl)benzol umgesetzt. Erhalten wurde eine klare, leicht gelbliche Flüssigkeit mit einer Viskosität von 100 mPa·s bei 20 °C, einem Amingehalt von 6.37 mmol N/g und einem theoretischen AHEW von ca. 124 g/Eq.

### Amin-5 (Referenz): 1,3-Bis(n-octylaminomethyl)benzol

Auf die gleiche Weise wie für das Amin-1 beschrieben wurden 25.6 g (0.20 mol) 1-Oktanal und 13.6 g (0.10 mol) 1,3-Bis(aminomethyl)benzol umgesetzt. Erhalten wurde eine klare, leicht gelbliche Flüssigkeit mit einer Viskosität von 130 mPa·s bei 20 °C, einem Amingehalt von 5.45 mmol N/g und einem theoretischen AHEW von ca. 180.3 g/Eq.

**Amin-6 (Referenz):** N,N'-Bis(2-ethylhexyl)-1,5-diamino-2-methylpentan Auf die gleiche Weise wie für das Amin-1 beschrieben wurden 25.6 g (0.20 mol) 1-Oktanal und 11.6 g (0.10 mol) 1,5-Diamino-2-methylpentan (Dytek^{®} A von Invista) umgesetzt. Erhalten wurde eine klare, leicht gelbliche Flüssigkeit mit einer Viskosität von 140 mPa·s bei 20 °C, einem Amingehalt von 5.80 mmol N/g und einem theoretischen AHEW von ca. 170.3 g/Eq.

### 4. Herstellung von Härtern

Für jedes Beispiel wurden die in der Tabelle 1 angegebenen Inhaltsstoffe in den angegebenen Mengen (in Gewichtsteilen) mittels eines Zentrifugalmischers (SpeedMixer™ DAC 150, FlackTek Inc.) vermischt. 1 Stunde nach dem Vermischen wurde jeweils die Viskosität der Härter-Zusammenstzung bestimmt. Die Resultate sind in Tabelle 1 angegeben.

Bei ***HZ-1*** bis ***HZ-3*** handelt es sich um erfindungsgemässe Härter, bei ***Ref-1*** bis ***Ref-3*** um Vergleichsbeispiele.

**Tabelle 1: Zusammensetzungen, Viskositäten und AHEW der Härter HZ-1 bis HZ-3 und Ref-1 bis Ref-3.**

| **Beispiel** | ***HZ-1*** | ***HZ-2*** | ***HZ-3*** | ***Ref-1*** | ***Ref-2*** | ***Ref-3*** |
|---|---|---|---|---|---|---|
| | | | | | | |
| EP-Addukt 1 | 74.6 | - | - | 74.6 | - | - |
| EP-Addukt 2 | - | 79.6 | - | - | 79.6 | - |
| Aradur^{®} 3442 | - | - | 93.8 | - | - | 93.8 |
| **Amin-1** | 45.1 | 45.1 | 45.1 | - | - | - |
| **Amin-3** | - | - | - | 39.6 | 39.6 | 39.6 |
| | | | | | | |
| Viskosität [mPa·s] (20°C) | 790 | 1'240 | 1'170 | 1'650 | 2'570 | 1'730 |
| AHEW [g] | 119.6 | 126.9 | 138.8 | 114.2 | 121.5 | 133.4 |

Jeder Härter der Tabelle 1 enthält das Amin-1 bzw. das Amin-3 in einer solchen Menge, dass seine Aminwasserstoffe 25 % der gesamthaft im Härter vorhandenen Aminwasserstoffe ausmachen.

### 5. Herstellung von Härtern und Epoxidharz-Zusammensetzungen

Für jedes Beispiel wurden die in den Tabellen 2 bis 3 angegebenen Inhaltsstoffe in den angegebenen Mengen (in Gewichtsteilen) der Härter-Komponente mittels eines Zentrifugalmischers (SpeedMixer™ DAC 150, FlackTek Inc.) vermischt und unter Ausschluss von Feuchtigkeit aufbewahrt.

Ebenso wurden die in den Tabellen 2 bis 3 angegebenen Inhaltsstoffe der Harz-Komponente verarbeitet und aufbewahrt.

Anschliessend wurden die beiden Komponenten jeder Zusammensetzung mittels des Zentrifugalmischers zu einer homogenen Flüssigkeit verarbeitet und diese unverzüglich folgendermassen geprüft:
10 Minuten nach dem Vermischen wurde die Viskosität bei 20°C bestimmt **("Viskosität (10')").**

Ein erster Film wurde in einer Schichtdicke von 500 µm auf eine Glasplatte aufgezogen und dieser bei 23±1 °C und 50±5 % relativer Feuchtigkeit (= Normklima, im folgenden abgekürzt mit "NK") gelagert, beziehungsweise ausgehärtet. An diesem Film wurde die **Königshärte** (Pendelhärte nach König, gemessen nach DIN EN ISO 1522) nach 2 Tagen ("Königshärte (2d NK)") bzw. nach 4 Tagen ("Königsh. (NK) (4d)") bzw. nach 7 Tagen ("Königshärte (7d NK)") bzw. nach 4 Wochen ("Königshärte (4w NK)") bestimmt. Nach 4 Wochen wurde der Aspekt des Films beurteilt (in der Tabelle mit "Aspekt (NK)" bezeichnet). Als "schön" wurde dabei ein Film bezeichnet, welcher klar war und eine glänzende und nichtklebrige Oberfläche ohne Struktur aufwies. Als "Struktur" wird dabei jegliche Art von Zeichnung oder Muster auf der Oberfläche bezeichnet.

Ein zweiter Film wurde in einer Schichtdicke von 500 µm auf eine Glasplatte aufgezogen und dieser unmittelbar nach dem Applizieren während 7 Tagen bei 8 °C und 80 % relativer Feuchtigkeit und anschliessend während 3 Wochen im NK gelagert, beziehungsweise ausgehärtet. 24 Stunden nach der Applikation wurde ein Flaschendeckel aus Polypropylen auf den Film aufgesetzt, unter welchem ein feuchtes Schwämmchen platziert war. Nach weiteren 24 Stunden wurde das Schwämmchen und der Deckel entfernt und an einer neuen Stelle des Films platziert, wo es nach 24 Stunden wieder entfernt und neu platziert wurde, insgesamt 4 mal. Anschliessend wurde der Aspekt dieses Films beurteilt (in den Tabellen mit "Aspekt (8°/80%)" bezeichnet), auf die gleiche Weise wie für den Aspekt (NK) beschrieben. Dabei wurde jeweils auch die Anzahl Markierungen angegeben, die im Film durch das feuchte Schwämmchen und/oder den aufgesetzten Deckel sichtbar waren. Trat an der Markierung eine Verfärbung oder Trübung auf, so ist dies ebenfalls angegeben. An den so ausgehärteten Filmen wurde wiederum die Königshärte bestimmt, jeweils nach 7 Tagen bei 8 °C und 80 % relativer Feuchtigkeit ("Königshärte (7d kalt)"), dann nach weiteren 2 Tagen im NK ("Königshärte (+2d NK)") bzw. 7 Tagen im NK ("Königshärte (+7d NK)") bzw. 3 Wochen im NK ("Königshärte (+3w NK)"). Die Vergilbung wurde bestimmt an einem in einer Schichtdicke von 500 µm auf eine Glasplatte aufgezogenen und während 4 Wochen im Normklima ausgehärteten Film, welcher anschliessend während 3 Monaten auf einer gut besonnten Terrasse gelagert wurde. Die Stärke der Vergilbung wurde visuell beurteilt im Vergleich zu einer mit Alufolie abgedeckten Fläche des Films.

Die Resultate sind in den Tabellen 2 bis 3 angegeben.

Bei ***EZ-1*** bis ***EZ-4*** handelt es sich um erfindungsgemässe Beispiele, bei ***Ref-4*** bis ***Ref-11*** um Vergleichsbeispiele.

**Tabelle 2: Zusammensetzung und Eigenschaften von EZ-1, EZ-2 und Ref-4 bis Ref-8. "I." steht für "leicht"; "schw" steht für "schwach" ¹ Gaskamine^{®} 240**

| | | | ***Ref-4*** | ***EZ-1*** | ***EZ-2*** | ***Ref-5*** | ***Ref-6*** | ***Ref-7*** | ***Ref-8*** |
|---|---|---|---|---|---|---|---|---|---|
| **Harz-Komp.:** | | | | | | | | | |
| | Araldit^{®} GY-250 | | 167.2 | 167.2 | 167.2 | 167.2 | 167.2 | 167.2 | 167.2 |
| | Araldite^{®} DY-E | | 31.8 | 31.8 | 31.8 | 31.8 | 31.8 | 31.8 | 31.8 |
| **Härter-Komp.:** | | | | | | | | | |
| | EP-Addukt 1 | | 99.4 | 74.6 | 74.6 | 74.6 | 74.6 | 74.6 | 74.6 |
| | Amin | | **-** | **-1** 45.1 | **-2** 32.6 | **-3** 39.6 | **-4** 31.1 | **-5** 45.1 | **240** ¹ 25.8 |
| | Ancamine^{®} K54 | | 6.0 | 6.4 | 6.2 | 6.3 | 6.2 | 6.4 | 6.0 |
| | | | | | | | | | |
| Viskosität (10') [Pa·s] | | | 2.90 | 1.11 | 1.45 | 1.54 | 1.65 | 1.10 | 1.62 |
| Königshärte (2d NK) | | | 145 | 77 | 131 | 133 | 104 | 78 | 116 |
| | [s] | (4d NK) | 176 | 88 | 141 | 170 | 123 | 84 | 160 |
| | | (7d NK) | 196 | 88 | 142 | 172 | 130 | 87 | 176 |
| | | (4w NK) | 198 | 106 | 147 | 200 | 147 | 90 | 193 |
| | Aspekt (NK) | | schön | schön | schön | schön | trüb | I. trüb | schön |
| | Königshärte | (7d kalt) | 187 | 39 | 59 | 70 | 42 | 34 | 42 |
| | (8°/80%) | (+2d NK) | 161 | 75 | 125 | 147 | 87 | 67 | 66 |
| | [s] | (+7d NK) | 185 | 97 | 140 | 165 | 120 | 80 | 133 |
| | | (+3w NK) | 197 | 105 | 144 | 183 | 137 | 83 | 186 |
| Aspekt (8°/80%) | | | I. matt | schön | I. matt | schön | trüb, Belag | I. matt | schön |
| Anzahl Markiehrungen | | | 4 (schw) | keine | keine | keine | keine | 1 (schw) | 4 (schw) |
| Vergilbung | | | minim | minim | minim | deutlich | minim | minim | deutlich |

**Tabelle 3: Zusammensetzung und Eigenschaften von EZ-3, EZ-4 und Ref-9 bis Ref-11. "weiss" steht für eine weisse Verfärbung der Markierung; "schw." steht für "schwach"**

| | | **Beispiel** | ***Ref-9*** | ***EZ-3*** | ***Ref-10*** | ***EZ-4*** | ***Ref-11*** |
|---|---|---|---|---|---|---|---|
| **Harz-Komp.:** | | | | | | | |
| | Araldite^{®} GY-250 | | 167.2 | 167.2 | 167.2 | 167.2 | 167.2 |
| | Araldite^{®} DY-E | | 31.8 | 31.8 | 31.8 | 31.8 | 31.8 |
| **Härter-Komp.:** | | | | | | | |
| | EP-Addukt 1 | | - | - | 59.6 | 49.7 | 49.7 |
| | EP-Addukt 2 | | 106.1 | 79.6 | - | | |
| | Jeffamine^{®} RFD 270 | | - | - | 26.8 | 22.3 | 22.3 |
| | **Amin-1** | | - | 45.1 | - | 30.1 | - |
| | **Amin-6** | | - | - | - | - | 28.4 |
| | Ancamine^{®} K 54 | | 6.1 | 6.5 | 5.7 | 6.0 | 6.0 |
| | | | | | | | |
| Viskosität (10') [Pa·s] | | | 5.29 | 1.53 | 1.74 | 0.91 | 1.01 |
| Königshärte[s] | | (2d NK) | 218 | 102 | 160 | 120 | 105 |
| | | (4d NK) | 223 | 114 | 181 | 141 | 123 |
| | | (7d N K) | 232 | 120 | 207 | 151 | 140 |
| | | (4w NK) | 231 | 130 | 211 | 170 | 142 |
| Aspekt (NK) | | | schön | schön | schön | schön | schön |
| Königshärte | | (7d kalt) | 139 | 56 | 113 | 52 | 38 |
| (8°/80%) | | (+2d NK) | 207 | 106 | 178 | 91 | 83 |
| [s] | | (+7d NK) | 217 | 122 | 192 | 126 | 118 |
| | | (+3w NK) | 216 | 126 | 216 | 155 | 130 |
| Aspekt (8°/80%) | | | matt | I.matt | schön | schön | I.matt |
| Anzahl Markierungen | | | 4 (weiss) | 4 (schw.) | 1 (schw.) | 1 (schw.) | keine |

## Patentansprüche

1. Amin der Formel (I).

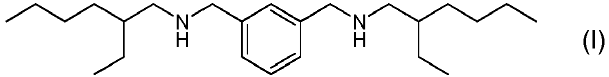

2. Verfahren zur Herstellung des Amins gemäss Anspruch 1 durch reduktive Alkylierung von 1,3-Bis(aminomethyl)benzol mit 2-Ethylhexanal und Wasserstoff.

3. Verwendung des Amins gemäss Anspruch 1 als Verdünner, insbesondere in Härtern für Epoxidharze.

4. Härter, geeignet zum Aushärten von Epoxidharzen, umfassend das Amin der Formel (I) gemäss Anspruch 1 und mindestens ein Polyamin **A,** welches mindestens drei gegenüber Epoxidgruppen reaktive Aminwasserstoffe aufweist.

5. Härter gemäss Anspruch 4, **dadurch gekennzeichnet, dass** das Polyamin **A** ausgewählt ist aus der Gruppe bestehend aus 1,5-Diamino-2-methyl-pentan, 2-Butyl-2-ethyl-1,5-pentandiamin, 1,6-Hexandiamin, 2,2,4- und 2,4,4-Trimethylhexamethylendiamin, 1,12-Dodecandiamin, 1,3-Diaminocyclohexan, Bis(4-aminocyclohexyl)methan , Bis(4-amino-3-methylcyclohexyl)methan, 1-Amino-3-aminomethyl-3,5,5-trimethylcyclohexan, 1,3-Bis(aminomethyl)cyclohexan, 1,3-Bis(aminomethyl)benzol, Bishexamethylentriamin, Diethylentriamin, Triethylentetramin, Tetraethylenpentamin, Pentaethylenhexamin und höhere Homologe linearer Polyethylenamine wie Polyethylenpolyamin mit 5 bis 7 Ethylenamin-Einheiten, Dipropylentriamin, N-(2-Aminoethyl)-1,3-propandiamin, N,N'-Bis(3-aminopropyl)ethylendiamin, cycloaliphatischen ethergruppenhaltigen Diaminen aus der Propoxylierung und nachfolgender Aminierung von 1,4-Dimethylolcyclohexan mit einem Molekulargewicht im Bereich von 200 bis 300 g/mol, Polyoxyalkylendiaminen und Polyoxyalkylentriaminen mit einem Molekulargewicht im Bereich von 200 bis 500 g/mol, sowie Derivaten dieser Polyamine in Form von Addukten mit Epoxiden oder in Form von Michael-Addukten, Mannich-Basen oder Polyamidoaminen.

6. Härter gemäss einem der Ansprüche 4 oder 5, **dadurch gekennzeichnet, dass** das Polyamin **A** ein Addukt mit einem Epoxid darstellt.

7. Härter gemäss einem der Ansprüche 4 bis 6, **dadurch gekennzeichnet, dass** das Polyamin **A** ein Addukt mit einem aromatischen Monoepoxid darstellt.

8. Härter gemäss einem der Ansprüche 4 bis 7, **dadurch gekennzeichnet, dass** das Amin der Formel (I) in einer solchen Menge vorliegt, dass seine Aminwasserstoffe 1 bis 75 % der gesamthaft im Härter vorhandenen Aminwasserstoffe ausmachen.

9. Härter gemäss einem der Ansprüche 4 bis 8, **dadurch gekennzeichnet, dass** das Amin der Formel (I) in einer solchen Menge vorliegt, dass sein Gewichtsanteil 1 bis 95 % der Summe aller gegenüber Epoxidgruppen reaktiven Amine ausmacht.

10. Härter gemäss einem der Ansprüche 4 bis 9, **dadurch gekennzeichnet, dass** der Gehalt an nicht einbaubaren Verdünnern weniger als 25 Gewichts-% beträgt.

11. Epoxidharz-Zusammensetzung enthaltend mindestens ein Epoxidharz und einen Härter gemäss einem der Ansprüche 4 bis 10.

12. Epoxidharz-Zusammensetzung gemäss Anspruch 11, **dadurch gekennzeichnet, dass** das Epoxidharz ein Flüssigharz auf der Basis eines Bisphenols ist.

13. Epoxidharz-Zusammensetzung gemäss einem der Ansprüche 11 und/oder 12, **dadurch gekennzeichnet, dass** sie eine zweikomponentige Zusammensetzung ist, bestehend aus
(i) einer Harz-Komponente enthaltend mindestens ein Epoxidharz und
(ii) einer Härter-Komponente enthaltend den Härter gemäss einem der Ansprüche 4 bis 10.

14. Ausgehärtete Zusammensetzung erhalten aus der Aushärtung einer Zusammensetzung gemäss einem der Ansprüche 11 bis 13.

15. Artikel enthaltend eine ausgehärtete Zusammensetzung gemäss Anspruch 14.

## Claims

1. An amine of formula (I).

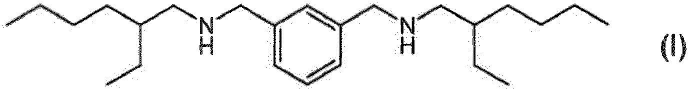

2. A process for producing the amine according to claim 1 by reductive alkylation of 1,3-bis-(aminomethyl)-benzene with 2-ethylhexanal and hydrogen.

3. Use of said amine according to claim 1 as a diluent, in particular in curing agents for epoxy resins.

4. A curing agent, suitable for curing epoxy resins, comprising the amine of formula (I) according to claim 1 and at least one polyamine **A** which has at least three epoxide group-reactive amine hydrogens.

5. The curing agent according to claim 4, **characterized in that** said polyamine **A** is selected from the group consisting of 1,5-diamino-2-methyl-pentane, 2-butyl-2-ethyl-1,5-pentanediamine, 1,6-hexanediamine, 2,2,4- and 2,4,4-trimethyl-hexamethylenediamine, 1,12-dodecanediamine, 1,3-diaminocyclohexane, bis-(4-aminocyclohexyl)-methane, bis-(4-amino-3-methylcyclohexyl)-methane, 1-amino-3-aminomethyl-3,5,5-trimethylcyclohexane, 1,3-bis-(aminomethyl)-cyclohexane, 1,3-bis-(aminomethyl)-benzene, bishexamethylenetriamine, diethylenetriamine, triethylenetetramine, tetraethylenepentamine, pentaethylenehexamine and higher homologs of linear polyethylene amines such as polyethylene polyamine having 5 to 7 ethylene amine units, dipropylenetriamine, N-(2-aminoethyl)-1,3-propanediamine, N,N'-bis-(3-aminopropyl)-ethylenediamine, cycloaliphatic ether group-containing diamines of the propoxylation and subsequent amination of 1,4-dimethylolcyclohexane having a molecular weight ranging from 200 to 300 g/mol, polyoxyalkylenediamines and polyoxyalkylenetriamines having a molecular weight ranging from 200 to 500 g/mol, and derivatives of these polyamines in the form of adducts with epoxides or in the form of Michael adducts, Mannich bases or polyamidoamines.

6. The curing agent according to any one of claims 4 or 5, **characterized in that** the polyamine **A** is an adduct with an epoxide.

7. The curing agent according to any one of claims 4 to 6, **characterized in that** the polyamine **A** is an adduct with an aromatic monoepoxide.

8. The curing agent according to any one of claims 4 to 7, **characterized in that** the amine of formula (I) is present in an amount such that its amine hydrogens constitute 1 to 75% of all amine hydrogens present in the curing agent.

9. The curing agent according to any one of claims 4 to 8, **characterized in that** the amine of formula (I) is present in an amount such that its proportion by weight is 1 to 95% of all epoxide group-reactive amines.

10. The curing agent according to any one of claims 4 to 9, **characterized in that** the content of non-incorporable diluents is less than 25% by weight.

11. The epoxy resin composition containing at least one epoxy resin and one curing agent according to any one of claims 4 to 10.

12. The epoxy resin composition according to claim 11, **characterized in that** the epoxy resin is a liquid resin on the basis of a bisphenol.

13. The epoxy resin composition according to any one of claims 11 and/or 12, **characterized in that** it is a two-component composition, consisting of
(i) a resin component containing at least one epoxy resin, and
(ii) a curing agent component containing the curing agent according to any one of claims 4 to 10.

14. A cured composition obtained from curing a composition according to any one of claims 11 to 13.

15. Articles containing a cured composition according to claim 14.

## Revendications

1. Amine de formule (I)

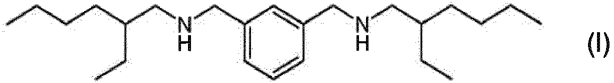

2. Procédé pour la préparation de l'amine selon la revendication 1 par alkylation réductrice de 1,3-bis(aminométhyl)benzène avec du 2-éthylhexanal et de l'hydrogène.

3. Utilisation de l'amine selon la revendication 1 comme diluant, en particulier dans des durcisseurs pour des résines époxyde.

4. Durcisseur approprié pour le durcissement de résines époxyde, comprenant l'amine de formule (I) selon la revendication 1 et au moins une polyamine A qui présente au moins trois hydrogènes d'amine réactifs par rapport aux groupes époxyde.

5. Durcisseur selon la revendication 4, **caractérisé en ce que** la polyamine A est choisie dans le groupe constitué par le 1,5-diamino-2-méthylpentane, la 2-butyl-2-éthyl-1,5-pentanediamine, la 1,6-hexanediamine, la 2,2,4-triméthylhexaméthylènediamine et la 2,4,4-triméthylhexaméthylènediamine, la 1,12-dodécanediamine, le 1,3-diaminocyclohexane, le bis(4-aminocyclohexyl)méthane, le bis(4-amino-3-méthylcyclohexyl)méthane, le 1-amino-3-aminométhyl-3,5,5-triméthylcyclohexane, le 1,3-bis(aminométhyl)cyclohexane, le 1,3-bis(aminométhyl)benzène, la bishexaméthylènetriamine, la diéthylènetriamine, la triéthylènetétraamine, la tétraéthylènepentaamine, la pentaéthylènehexaamine et des polyéthylèneamines linéaire homologues supérieures, telles que la polyéthylènepolyamine comprenant 5 à 7 unités d'éthylèneamine, la dipropylènetriamine, la N-(2-aminoéthyl)-1,3-propanediamine, la N,N'-bis(3-aminopropyl)éthylènediamine, les diamines cycloaliphatiques contenant des groupes éther, provenant de la propoxylation et de l'amination consécutive de 1,4-diméthylolcyclohexane présentant un poids moléculaire dans la plage de 200 à 300 g/mole, les polyoxyalkylènediamines et les polyoxyalkylènetriamines présentant un poids moléculaire dans la plage de 200 à 500 g/mole, ainsi que les dérivés de ces polyamines sous forme de produits d'addition avec des époxydes ou sous forme de produits d'addition de Michael, de bases de Mannich ou de polyamidoamines.

6. Durcisseur selon l'une quelconque des revendications 4 ou 5, **caractérisé en ce que** la polyamine A représente un produit d'addition avec un époxyde.

7. Durcisseur selon l'une quelconque des revendications 4 à 6, **caractérisé en ce que** la polyamine A représente un produit d'addition avec un monoépoxyde aromatique.

8. Durcisseur selon l'une quelconque des revendications 4 à 7, **caractérisé en ce que** l'amine de formule (I) se trouve en une quantité telle que ses hydrogènes d'amine représentent 1 à 75% de la totalité des hydrogènes d'amine se trouvant dans le durcisseur.

9. Durcisseur selon l'une quelconque des revendications 4 à 8, **caractérisé en ce que** l'amine de formule (I) se trouve en une quantité telle que sa proportion pondérale représente 1 à 95% de la somme de toutes les amines réactives par rapport aux groupes époxyde.

10. Durcisseur selon l'une quelconque des revendications 4 à 9, **caractérisé en ce que** la teneur en diluants non incorporables est inférieure à 25% en poids.

11. Composition de résine époxyde contenant au moins une résine époxyde et un durcisseur selon l'une quelconque des revendications 4 à 10.

12. Composition de résine époxyde selon la revendication 11, **caractérisée en ce que** la résine époxyde est une résine liquide à base d'un bisphénol.

13. Composition de résine époxyde selon l'une quelconque des revendications 11 et/ou 12, **caractérisée en ce qu'**il s'agit d'une composition à deux composants, constituée par
(i) un composant de résine, contenant au moins une résine époxyde et
(ii) un composant durcisseur, contenant le durcisseur selon l'une quelconque des revendications 4 à 10.

14. Composition durcie obtenue à partir du durcissement d'une composition selon l'une quelconque des revendications 11 à 13.

15. Objet, contenant une composition durcie selon la revendication 14.
